# EUROPEAN PATENT APPLICATION

(11) **EP 3 431 090 A1**
(43) Date of publication of application: **23.01.2019**
(21) Application number: 17766646.8
(22) Date of filing: 14.03.2017
(51) Int. Cl.: A61K 31/702, A23L 33/135, A61P 1/04, C12N 1/20

(54) **PROLIFERATIVE AGENT FOR BACTERIA OF GENUS FAECALIBACTERIUM**

(30) Priority: 14.03.2016 JP 2016049586
(71) Applicant: B Food Science Co., Ltd., Chita-shi Aichi 478-0046 (JP)
(72) Inventor: TOCHIO, Takumi, Chita-shi Aichi 478-0046 (JP); SATO, Fuyuhiko, Chita-shi Aichi 478-0046 (JP); KONISHI, Kenta, Chita-shi Aichi 478-0046 (JP); KOGA, Yasuhiro, Isehara-shi Kanagawa 259-1143 (JP); ENDO, Akihito, Tokyo 156-8502 (JP)
(74) Representative: Wasner, Marita
(86) International application number: PCT/JP2017/010070
(87) International publication number: WO 2017/159647

(57) **Abstract**

[Problem]

To provide a substance and method that are capable of effectively inducing proliferation of Faecalibacterium, thereby contributing to the prevention and treatment of diseases such as intestinal inflammation.

[Solution]

A proliferative agent for Faecalibacterium, comprising 1-kestose as an active ingredient. In accordance with the present invention, proliferation of Faecalibacterium can be effectively induced both in vitro and in vivo. In particular, it is possible to simply and effectively increase in vivo proliferation of Faecalibacterium in humans and animals, with almost no concerns regarding side effects or safety. It is also possible to prevent or treat intestinal inflammation by increasing in vivo proliferation of Faecalibacterium in humans and animals.

## Description

### Technical Field

The present invention relates to a proliferative agent for Faecalibacterium comprising 1-kestose as an active ingredient, a food composition for proliferating Faecalibacterium, a method for proliferating Faecalibacterium, a method for treatment or prevention of intestinal inflammation, and the use of 1-kestose for producing a pharmaceutical preparation for treatment or prevention of intestinal inflammation.

### Background Art

Bacteria of the genus Faecalibacterium are obligate anaerobic bacilli that reside in the intestinal mucus layer and main bacterial constituents of the intestinal bacterial flora and the number and percentage of their presence in humans is high. Recent studies have demonstrated that Faecalibacterium have preferable physiological functions such as supplying energy to intestinal cells, maintaining a good intestinal environment, and a strong anti-inflammatory function (Non Patent Literature 1). Furthermore, it has been reported about the anti-inflammatory function that the inflammatory state can be prevented or improved by administering Faecalibacterium or a culture supernatant thereof to enteritis model animals and cultured cells (Non Patent Literatures 2 and 3).

Meanwhile, methods of administering Faecalibacterium as such described in Non Patent Literature 1 and 2 to the living body for the purpose of the prevention or treatment of a disease or health promotion have problems such as the following (i) to (iv): (i) In the case of oral administration, most of the bacteria are subjected to the actions of gastric acid, bile, and digestive juices and die before reaching the intestine. (ii) Since exogenous bacteria are generally less likely to colonize in the intestine and the majority pass through the gastrointestinal tract, their effects are considered to be transient. (iii) Since there is no history of eating Faecalibacterium, there is a concern for safety. (iv) Since Faecalibacterium are obligate anaerobic, special processing or storage conditions are needed to incorporate them into pharmaceutical preparations and foods as living bacteria.

Accordingly, in order to solve the problems of (i) to (iv), attempts to increase endogenous Faecalibacterium that reside in the living body have been made. For example, Patent Literature 1 discloses a method of ingesting a composition comprising polydextrose or soluble corn fiber to increase Faecalibacterium.

### Citation List

### Patent Literature

Patent Literature 1: Japanese Patent Laid Open No. 2014-532710

### Non Patent Literature

Non Patent Literature 1: Yuan Gao et. al., Gastroenterology Research and Practice, Vol.2014, Art.ID 872725, March 27, 2014
Non Patent Literature 2: Miquel S. et. al., Current Opinion in Microbiology, Vol.16 (3), p. 255-261, June 2013
Non Patent Literature 3: Xinyun Qiu et. al., Journal of Crohn's and Colitis, Vol.7, Issue 11, e558-e568, December 2013

### Summary of the Invention

### Technical Problem

However, no substance or method capable of sufficiently increasing the number of Faecalibacterium has been sufficiently provided and the development of such a substance or method has been desired. The present invention was made to solve the problem and an object of the present invention is to provide a substance or a method that can effectively proliferate Faecalibacterium and thereby contribute to prevention and treatment of a disease such as intestinal inflammation.

### Solution to the Problem

The present inventors have found, as a result of diligent study, that 1-kestose markedly increases the number of Faecalibacterium both in vitro and in vivo. Accordingly, based on these findings, the following inventions were completed.
(1) The proliferative agent for Faecalibacterium according to the present invention comprises 1-kestose as an active ingredient.
(2) The proliferative agent for Faecalibacterium according to the present invention can be used for the prevention or treatment of intestinal inflammation.
(3) The proliferative agent for Faecalibacterium according to the present invention can preferably be used for the prevention or treatment of intestinal inflammations, in particular, inflammatory bowel disease.
(4) The proliferative agent for Faecalibacterium according to the present invention can preferably be used for prevention or treatment of inflammatory bowel diseases, in particular, Crohn disease or ulcerative colitis.
(5) A food composition for proliferating Faecalibacterium according to the present invention comprises 1-kestose as an active ingredient.
(6) The first aspect of the method for proliferating Faecalibacterium according to the present invention comprises the step of increasing the number of Faecalibacterium in intestines in a human or an animal by letting the human or the animal take 1-kestose.
(7) The second aspect of the method for proliferating Faecalibacterium according to the present invention comprises the step of adding 1-kestose to a medium containing Faecalibacterium to culture the Faecalibacterium.
(8) The method for treatment or prevention of intestinal inflammation comprises the step of increasing the number of Faecalibacterium in intestines in a human or an animal having or being at risk of having intestinal inflammation by letting the human or the animal take 1-kestose.
(9) The use of 1-kestose according to the present invention is use of 1-kestose for producing a pharmaceutical preparation for treatment or prevention of intestinal inflammation.

### Advantageous Effects of Invention

According to the present invention, Faecalibacterium can be effectively proliferated both in vitro and in vivo.

Moreover, since 1-kestose is an oligosaccharide which is also contained in vegetables such as onion and garlic, and in cereals such as barley and rye, and as it is a substance that has been taken as foods since ancient times, and since no toxicity of 1-kestose has been found in any of mutagenicity, acute toxicity, subchronic toxicity and chronic toxicity tests, it is considered to be very safe (Food processing and ingredients, Vol. 49, No. 12, p. 9, 2014).
Moreover, since 1-kestose is highly water-soluble and has a good sweet taste quality similar to sugar, it can be taken easily on a daily basis as it is or as a sweetener or the like, and easily be formulated into various foods, pharmaceutical preparations and the like.

Accordingly, Faecalibacterium in human and animal bodies can be easily and effectively increased with little concerns about side effects and safety, according to the present invention. Moreover, according to the present invention, intestinal inflammation can be prevented or treated by increasing Faecalibacterium in human and animal bodies.

### Brief Description of the Drawings

[Figure 1] Figure 1 is a line graph illustrating the relation between the turbidity (OD value) and culture time of Faecalibacterium prausnitzii strain ATCC27768 cultured with a variety of oligosaccharides.
[Figure 2] Figure 2 is a bar graph illustrating the turbidity (OD value) of the culture of Faecalibacterium prausnitzii strain ATCC27768 cultured with a variety of oligosaccharides for 72 hours.

### Description of Embodiments

A proliferative agent for Faecalibacterium, a food composition for proliferating Faecalibacterium, a method for proliferating Faecalibacterium, a method for treatment or prevention of intestinal inflammation, and the use of 1-kestose for producing a pharmaceutical preparation for treatment or prevention of intestinal inflammation according to the present invention will be described in detail below.

In the present invention, "Faecalibacterium" are microorganisms belonging to the genus Faecalibacterium. An example of the microorganisms belonging to the genus Faecalibacterium is Faecalibacterium prausnitzii.

"1-Kestose" is an oligosaccharide that is a trisaccharide composed of 1 molecule of glucose and 2 molecules of fructose. 1-Kestose can be produced by an enzymatic reaction of sucrose as a substrate with an enzyme such as one disclosed in Japanese Patent Laid Open No. 58-201980. Specifically, β-fructofuranosidase is added to a sucrose solution and the enzymatic reaction is performed by leaving the mixture at 37°C to 50°C for around 20 hours to obtain a reaction solution containing 1-kestose. This reaction solution containing 1-kestose is purified by separating 1-kestose and other sugars (glucose, fructose, sucrose, oligosaccharides composed of 4 or more sugars) using the chromatographic separation disclosed in Japanese Patent Laid Open No. 2000-232878 to obtain a high purity 1-kestose solution. 1-kestose can be obtained as crystals by subsequently concentrating this high purity 1-kestose solution and then crystallizing 1-kestose by a crystallization process such as one disclosed in Japanese Patent Publication No. 6-70075.

Moreover, 1-kestose is contained in commercially available fructo-oligosaccharides and therefore these may be used as they are or after separating and purifying 1-kestose from the fructo-oligosaccharides by the aforementioned method. Accordingly, 1-kestose-containing compositions, such as oligosaccharides containing 1-kestose, may be used as the 1-kestose of the present invention. When a 1-kestose-containing composition is used, the purity of the 1-kestose is preferably 80% by mass or more, more preferably 85% by mass or more, and further preferably 90% by mass or more. The "purity" of a certain oligosaccharide in the present invention is percent by mass of the oligosaccharide when taking the total amount of the sugars as 100%.

1-kestose can be used by letting a human or an animal take the 1-kestose. Examples of the intake (dose) of 1-kestose include 0.01 to 0.34 g/kg body weight, preferably 0.01 to 0.30 g/kg body weight, and more preferably 0.01 to 0.24 g/kg body weight. Such an intake may be taken once a day or divided into multiple doses.

1-Kestose has the function of increasing the number of Faecalibacterium in vivo as shown in Example 2 described later. Accordingly, the method for letting a human or an animal take 1-kestose may be any method, as long as it is a method that allows 1-kestose to reach the intestine, where Faecalibacterium resides. Specific examples of the method include letting a human or an animal take 1-kestose as it is or in the form of beverage or food or a pharmaceutical preparation. Other examples include administering 1-kestose from anus directly or through an inserted tube, adding 1-kestose to an enteral nutrition agent and administering that by enteral nutrition via a tube inserted into a gastrointestinal tract such as the stomach or the small intestine, and the like.

On the other hand, 1-kestose can be also used to increase the number of Faecalibacterium in vitro as shown in Example 1 described later. In this case, for example, a method adding 1-kestose to a medium containing Faecalibacterium to culture statically at 37°C for a predetermined time in an anaerobic environment is available.

As described above, it has been shown for Faecalibacterium that an inflammatory state can be prevented or improved by administration of the cells or a culture supernatant thereof. More specifically, Non Patent Literature 2 discloses that enteritis can be suppressed by administering Faecalibacterium prausnitzii strain A2-165 or a culture supernatant thereof to a model mouse having enteritis induced by 2,4,6-trinitrobenzenesulfonic acid (TNBS). Moreover, Non Patent Literature 3 discloses that the addition of Faecalibacterium prausnitzii (ATCC27766) and culture supernatant to a culture of human peripheral blood mononuclear cells can promote the production of anti-inflammatory cytokine such as IL-10, TGF-β1, and IL-12p70 and promote the differentiation of regulatory T cells. Furthermore, Non Patent Literature 3 also discloses that oral administration of Faecalibacterium prausnitzii (ATCC27766) or a culture supernatant thereof to a model rat having enteritis induced by TNBS can promote healing of inflammatory tissue found on intestinal mucosa, in addition to the promotion of production of anti-inflammatory cytokines and differentiation of regulatory T cells.

Accordingly, intestinal inflammation can be prevented or treated by letting a human or an animal take 1-kestose and increasing the number of Faecalibacterium in the body or the intestine thereof. Moreover, 1-kestose can be used to produce pharmaceutical preparations for treatment or prevention of intestinal inflammation.

Here, "intestinal inflammation" in the present invention means conditions with inflammation in the intestinal tract and includes disease conditions. Examples of diseases developed from intestinal inflammation include inflammatory bowel disease. The term inflammatory bowel disease may narrowly refer ulcerative colitis and Crohn disease or generally refer any enteric inflammatory disease (Integrated handbook of internal medicine (Progress 8), Gastrointestinal Diseases, pp. 320, 1997, Nakayama Shoten Co., Ltd.). The inflammatory bowel disease in the present invention means the latter, namely any inflammatory disease in an intestinal tract. The present invention may preferably be used for the prevention or treatment of intestinal inflammation excluding allergy.

Examples of specific aspects of the proliferative agent for Faecalibacterium according to the present invention include pharmaceutical preparations and quasi drugs, food additives, and health foods such as supplements.

The dosage form of the pharmaceutical preparations, quasi drugs, and supplements containing 1-kestose are not particularly limited, and a dosage form suitable for the mode of administration can be selected as appropriate. For example, when orally administered, the dosage form may be a solid or liquid dosage form such as a powder, a tablet, a dragee, a capsule, a granule, a dry syrup, a solution, a syrup, a drop, and a health drink.

The aforementioned dosage forms of pharmaceutical preparations, quasi drugs, and supplements can be prepared by methods known to those skilled in the art. For example, for a powder, 800 g of 1-kestose and 200 g of lactose are mixed well and then the mixture is wetted by adding 300 mL of 90% ethanol. Subsequently, the wet powder is granulated, dried with ventilation at 60°C for 16 hours, and then sized to obtain 1000 g of a suitable size of powder (1-kestose content 800 mg/1 g). For tablets, 300 g of 1-kestose, 380 g of powdery reduced starch syrup, 180 g of rice starch, and 100 g of dextrin are mixed well and then the mixture is wetted by adding 300 mL of 90% ethanol. Subsequently, the wet powder is extruded and granulated and then dried with ventilation at 60°C for 16 hours to obtain granules. Then, these granules were sized using a 850 µm sieve, followed by adding 50 g of a sucrose fatty acid ester and mixing with 470 g of the sized granules, and then the mixture was pressed into tablets with a rotary tableting machine (6B-2, manufactured by Kikusui Seisakusho Ltd.) to obtain 5000 tablets having a diameter of 8 mm and a weight of 200 mg (1-kestose content 60 mg/tablet).

Specific embodiments of the food composition for proliferating Faecalibacterium according to the present invention include, for example, beverages, dairy products, granules for eating, pastes, flavoring agents, retort pouches, baby foods, fermented foods, preserved foods, processed foods such as processed marine products, processed meat products, and processed grain products, food additives, health foods, and animal feed.

1-kestose can be used by being added to various foods and drinks, food additives, and animal feed in their normal production process. Since 1-kestose has a degree of sweetness of 30 and its quality of taste, physical properties, and workability are close to those of sucrose, it can be used like sugar in the production process of various foods and drinks, for example, by replacing a part or all sugar with 1-kestose, and various foods and drinks, food additives, and animal feed can be produced.

The present invention will be described with reference to the Examples below. The technical scope of the present invention is not limited by the features illustrated by these Examples. In the following Examples, compositions containing certain oligosaccharides at predetermined purities are described with the names of the oligosaccharides.

### Examples

### <Example 1> Study on Faecalibacterium proliferative effect in vitro

Liquid media of the following composition was prepared sterile. 1-Kestose (purity of 99% by mass, B Food Science Co., Ltd.), nystose (purity of 98% by mass, Wako Pure Chemical Industries, Ltd.), xylooligosaccharide (purity of 99% by mass, B Food Science Co., Ltd.), raffinose (purity of 98% by mass, Meiji Food Materia Co., Ltd.) and lactosucrose (purity of 89% by mass, Ensuiko Sugar Refining Co., Ltd.) were used as oligosaccharides. Moreover, a liquid medium of the following composition with no oligosaccharide was also prepared as a control.

### [Composition of liquid medium/per L] (final pH: 7.0)

Casein digest 15.0 g, yeast extract 5.0 g, L-cystine 0.5 g, sodium chloride 2.5 g, thioglycolic acid 0.5 g, tryptophan 25 mg, hemin proper quantity, vitamin K1 proper quantity, oligosaccharide 5.0 g.

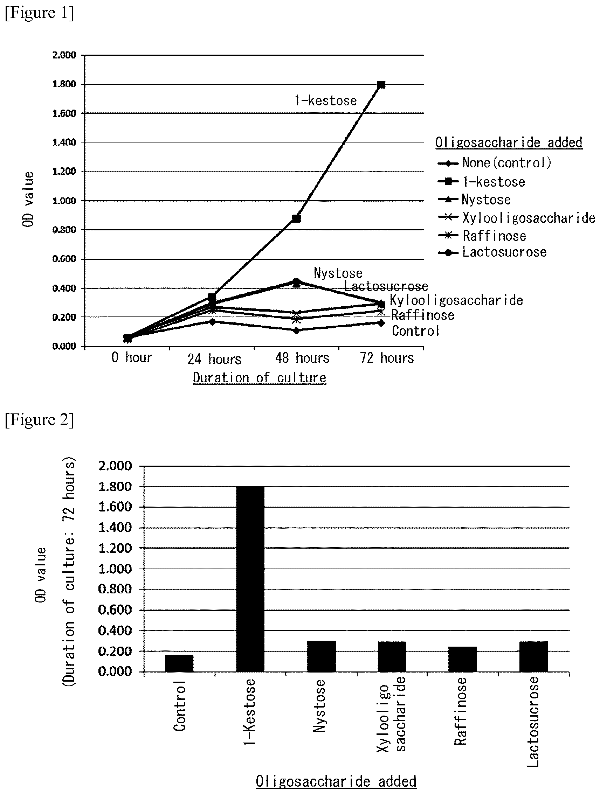

A prepared liquid medium was inoculated with Faecalibacterium prausnitzii strain ATCC27768 precultured until confluent to a turbidity with an OD (Optical Density) value at 660 nm of 0.05 to 0.06. The inoculated culture was cultured statically at 37°C in anaerobic environment and the turbidity (OD value) at 660 nm was measured with a spectrophotometer after 24 hours, 48 hours, and 72 hours. The result is shown in Table 1. Moreover, the relation between OD value and the duration of culture is illustrated in the line graph of Figure 1. Moreover, the OD values for the culture time of 72 hours are illustrated in a bar graph of Figure 2.

**[Table 1]**

| | Duration of culture | | | |
|---|---|---|---|---|
| Oligosaccharide added | 0 hour | 24 hours | 48 hours | 72 hours |
| None (control) | 0.062 | 0.173 | 0.113 | 0.166 |
| 1-kestose | 0.063 | 0.345 | 0.885 | 1.803 |
| Nystose | 0.063 | 0.290 | 0.443 | 0.305 |
| Xyrooligosaccharide | 0.069 | 0.271 | 0.234 | 0.294 |
| Raffinose | 0.052 | 0.251 | 0.191 | 0.246 |
| Lactosucrose | 0.055 | 0.298 | 0.448 | 0.295 |

As shown in Table 1, Figure 1, and Figure 2, while the OD values of the liquid medium of the control were 0.062 at 0 hours, 0.173 at 24 hours, 0.113 at 48 hours, and 0.166 at 72 hours; the OD values of the liquid medium containing 1-kestose were 0.063 at 0 hours, 0.345 at 24 hours, 0.885 at 48 hours, and 1.803 at 72 hours. The OD values of the liquid medium containing nystose were 0.063 at 0 hours, 0.290 at 24 hours, 0.443 at 48 hours, and 0.305 at 72 hours. The OD values of the liquid medium containing xylooligo saccharide were 0.069 at 0 hours, 0.271 at 24 hours, 0.234 at 48 hours, and 0.294 at 72 hours. The OD values of the liquid medium containing raffinose were 0.052 at 0 hours, 0.251 at 24 hours, 0.191 at 48 hours, and 0.246 at 72 hours. The OD values of the liquid medium with lactosucrose were 0.055 at 0 hours, 0.298 at 24 hours, 0.448 at 48 hours, and 0.295 at 72 hours.

Accordingly, the OD values of the liquid medium with 1-kestose were markedly greater at any duration of culture of 24 hours, 48 hours, and 72 hours, in comparison with the liquid medium containing no oligosaccharide (control liquid culture) and the liquid medium with other oligosaccharides (nystose, xylooligo saccharide, raffinose, and lactosucrose).

The turbidity (OD value) of the culture reflects the number of the microorganisms. Accordingly, from the results described above, the number of cells of Faecalibacterium prausnitzii strain ATCC27768 in the liquid medium containing 1-kestose was found to be markedly greater at any duration of culture of 24 hours, 48 hours, and 72 hours in comparison with the number of cells in the liquid medium containing no oligosaccharide and the liquid medium containing other oligosaccharides. From this result, 1-kestose was found to have a greater proliferative effect on Faecalibacterium in vitro.

### <Example 2> Study on the Faecalibacterium proliferative effect in vivo

58 infants from 6 months old to 6 years old were divided into 2 groups of 29 infants and they were designated 1-Kestose Group and Maltose Group. The 1-Kestose Group took 1-kestose (purity 98% by mass or more, B Food Science Co., Ltd.) orally and the Maltose Group took maltose (purity of 92% by mass or more, "Sunmalt-S" powder, Sanwa Starch Co., Ltd.) orally every day during the study period. The test period was 12 weeks. The daily intake of 1-kestose or maltose was 1 g (estimated intake; 0.121 g/kg body weight/day) for infants under 1 year old, 2 g (estimated intake; 0.169 g/kg body weight/day) for infants from 1 to 3 years old, and 3 g (estimated intake; 0.170 g/kg body weight/day) for infants from 4 to 6 years old. Feces were collected at the time of the start of the study period (0 weeks), at 6 weeks from the start (6 weeks), and at 12 weeks after the start (12 weeks).

The estimated intake mentioned above was calculated based on the age-specific body weight data published in "2010 National growth survey on preschool children of the Ministry of Health, Labour and Welfare". More specifically, the male and female medians were separately calculated from the minimums and the maximums of the body weight data within the age ranges of the study participants of Example 2 and the male and female mean values of the medians were separately calculated Subsequently, the mean value of the median was calculated based on the male and female mean values, and this was taken as the reference weight. The reference weights were 8.26 kg for under 1 year old, 11.85 kg for 1 to 3 years old, and 17.62 kg for 4 to 6 years old. The daily intake of 1-kestose or maltose was divided by this reference weight to obtain a reference intake.

The numbers of cells of Faecalibacterium in the recovered feces were quantified by real-time PCR. Specifically, genomic DNA was first extracted from the feces using the reagent for genome extraction "Ultra Clean Soil DNA Isolation Kit" (Mo Bio Laboratories Inc.) and "High Pure PCR Template Preparation Kit" (Roche). Real-time PCR was performed using this genomic DNA as a template, primers specific for Faecalibacterium (whose sequences were shown below), the real-time PCR reagent "SYBR GREEN Master Mix" (Applied Biosystems), and a real-time PCR device "ABI PRISM7700 sequence detection system" (Applied Biosystems) and the numbers of cells of Faecalibacterium per 1 g of feces were quantified. The detection range was equal to or greater than 10⁵ cells/g and the value 10^{2.5} cells/g was substituted for the samples less than detection range. The standard curve was generated based on the result of real-time PCR performed under the same conditions using genomic DNA extracted in the same way from Faecalibacterium prausnitzii strain ATCC27768 cultured in pure culture and counted under a microscope.

### [Sequences of primers specific for Faecalibacterium]

Forward primer; CCATGAATTGCCTTCAAAACTGTT (SEQ ID NO: 1)
Reverse primer; GAGCCTCAGCGTCAGTTGGT (SEQ ID NO: 2)

The mean of cell numbers was calculated for each group. Moreover, significant difference of the mean of cell numbers at 6 weeks and at 12 weeks from the mean of cell numbers at 0 weeks was tested by the Wilcoxon signed rank test and p values were determined. The results are shown in Table 2.

**[Table 2]**

| **1-kestose group** | | | |
|---|---|---|---|
| | 0 week | 6 weeks | 12 weeks |
| (cells/g feces) | **3.7 × 10⁶** | **1.4 × 10⁷** | **3.5 × 10⁷** |
| (cells log10/g feces) | **6.57** | **7.16** | **7.54** |
| Significant difference (vs 0 week) | - | Not found (p≒0.130) | Found (p≒0.026) |

| **Maltose group** | | | |
|---|---|---|---|
| | 0 week | 6 weeks | 12 weeks |
| (cells/g feces) | **7.6 × 10⁶** | **5.8 × 10⁶** | **1.3 × 10⁷** |
| (cells log10/g feces) | **6.88** | **6.77** | **7.12** |
| Significant difference (vs 0 week) | - | Not found (p≒0.627) | Not found (p≒0.614) |

As shown in Table 2, while the cell number of 1-Kestose Group was 3.7 x 10⁶ cells/g feces at 0 weeks, the cell number was 1.4 x 10⁷ cells/g feces at 6 weeks and 3.5 x 10⁷ cells/g feces at 12 weeks. Thus, the number of bacteria compared to 0 weeks increased to 3.5 times or more in 6 weeks, although there was no significant difference, and significantly increased 9 times or more in 12 weeks.

On the other hand, while the cell number of the Maltose Group was 7.6 x 10⁶ cells/g feces at 0 weeks, the cell number was 5.8 x 10⁶ cells/g feces at 6 weeks and 1.3 x 10⁷ cells/g feces at 12 weeks. Thus, the cell number at 6 weeks was slightly decreased and the cell number at 12 weeks was slightly increased, but not significantly different, from the cell number at 0 weeks.

As seen above, it was shown that the cell number of Faecalibacterium increased markedly in the 1-Kestose Group while the cell number did not increase in the Maltose Group. This result has revealed that 1-kestose has a markedly large proliferative effect on Faecalibacterium in vivo.

## Claims

1. A proliferative agent for Faecalibacterium, comprising 1-kestose as an active ingredient.

2. The proliferative agent for Faecalibacterium according to claim 1, wherein the agent is used for prevention or treatment of intestinal inflammation.

3. The proliferative agent for Faecalibacterium according to claim 2, wherein the intestinal inflammation is inflammatory bowel disease.

4. The proliferative agent for Faecalibacterium according to claim 3, wherein the inflammatory bowel disease is Crohn disease or ulcerative colitis.

5. A food composition for proliferating Faecalibacterium, comprising 1-kestose as an active ingredient.

6. A method for proliferating Faecalibacterium, comprising a step of increasing the number of Faecalibacterium in intestines in a human or an animal by letting the human or the animal take 1-kestose.

7. A method for proliferating Faecalibacterium, comprising a step of adding 1-kestose to a medium containing Faecalibacterium to culture Faecalibacterium.

8. A method for treatment or prevention of intestinal inflammation, comprising a step of increasing the number of Faecalibacterium in intestines in a human or an animal having or being at risk of having intestinal inflammation by letting the human or the animal take 1-kestose.

9. Use of 1-kestose for producing a pharmaceutical preparation for treatment or prevention of intestinal inflammation.


SEQUENCE LISTING

<110>  B Food Science Co., Ltd.
       Tokai medical test laboratory CO.

<120>  Faecalibacterium proliferator

<130>  PCT0726

<150>  JP2016-049586
<151>  2016-03-14

<160>  2

<170>  PatentIn version 3.5

<210>  1
<211>  24
<212>  DNA
<213>  Artificial Sequence

<220>
<223>  forward primer

<400>  1
ccatgaattg ccttcaaaac tgtt                                              24


<210>  2
<211>  20
<212>  DNA
<213>  Artificial Sequence

<220>
<223>  reverse primer

<400>  2
gagcctcagc gtcagttggt                                                   20